# EUROPEAN PATENT APPLICATION

(11) **EP 4 095 267 A1**
(43) Date of publication of application: **30.11.2022**
(21) Application number: 21175843.8
(22) Date of filing: 26.05.2021
(51) Int. Cl.: C12Q 1/6886

(54) **METHOD AND SYSTEM FOR DETERMINING EFFICACY OF CANCER THERAPY**

(71) Applicant: Siemens Healthcare GmbH, 91052 Erlangen (DE)
(72) Inventor: DIETRICH, Carsten, 90429 Nürnberg (DE); GEORGI, Jens-Christoph, 90522 Oberasbach (DE); KHANDIGE, Divya, 560032 Bangalore, Karnataka (IN); KOLLEGAL, Manohar G, 560102 Bangalore, Karnataka (IN); MITRA, Nivedita, 560066 Bangalore, Karnataka (IN); RAMYA, V M, 560019 Bangalore, Karnataka (IN)
(74) Representative: Horn Kleimann Waitzhofer Patentanwälte PartG mbB

(57) **Abstract**

A method (200) and a system (100) for determining efficacy of a cancer therapy in a patient is disclosed. In one aspect, the method (200) includes determining a first amount of circulating tumor nucleic acid present in the patient before initiating at least one instance of the cancer therapy. The method (200) further includes determining a second amount of circulating tumor nucleic acid present in the patient after the at least one instance of the cancer therapy. Additionally, the method (200) includes computing a difference between the first amount of circulating tumor nucleic acid and the second amount of circulating tumor nucleic acid. Furthermore, the method (200) includes determining the efficacy of the cancer therapy based on the computed difference.

## Description

The present invention relates to a method and system to determine efficacy of cancer therapy.

Effective and quick detection of clinically relevant analytes is the need of the hour. Circulating tumor nucleic acid acts as a marker of presence of cancer in a patient. The levels of circulating tumor nucleic acid in the patient's body indicates the presence of a tumor. Therefore, measuring levels of circulating tumor nucleic acid at different stages of cancer detection, therapy and monitoring is useful in understanding the course of therapy that the patient may undergo.

When a patient undergoes a cancer therapy, it is usually uncertain if the cancer cells in the patient's body will undergo cell death in response to the treatment. Therefore, early diagnosis of effectiveness of any therapy a patient undergoes is important. This enables the treatment plan to be adapted according to receptivity of the patient such that a desired effect of the therapy is achieved. Early identification of the patient's response to the therapy also prevents significant side effects that may be caused to the patient in addition to saving time and cost. Currently, systematic monitoring treatment effects often depends on the expertise of the physician. Additionally, effects of treatment of the patient may also be monitored by measuring the tumor size with classical anatomical imaging, such as Response Evaluation Criteria in Solid Tumors (RECIST) analysis with computed tomography imaging. However, such methods of identification of treatment efficacy is relatively late, i.e., when the patient has already undergone half of the treatment.

Currently, there is no way in which a physician can be informed if a patient is responding to a treatment, early on during the treatment.

Therefore, there exists a need for a method that enables early recognition of patient's response to a cancer therapy/treatment.

The object of the invention is therefore to provide a method and a system that enables determination of efficacy of cancer therapy early on during a treatment process.

The invention achieves the object by a method of determining efficacy of a cancer therapy in a patient. The method comprises determining a first amount of circulating tumor nucleic acid present in the patient before initiating at least one instance of the cancer therapy. Circulating tumor nucleic acid is a marker found in blood of the patient diagnosed with cancer. Circulating tumor nucleic acid may originate from cancerous cells or tumor(s) in the patient's body. This may include, but not be limited to, circulating tumor DNA, micro RNA (miRNA) and/or long non-coding RNA (IncRNA). The circulating tumor nucleic acid may also be obtained from patient samples such as serum, plasma, lymph, urine, saliva and other body fluids. Determining the first amount of circulating tumor nucleic acid present in the patient enables setting a baseline using which efficacy of the cancer therapy may be determined. The method further comprises subjecting the patient to the at least one instance of cancer therapy. The cancer therapy may include, for example, chemotherapy, radiation therapy, surgery, etc. This may be determined based on a type of cancer that the patient may be diagnosed with.

The method further comprises determining a second amount of circulating tumor nucleic acid present in the patient after initiating the at least one instance of the cancer therapy. In an embodiment, the first amount and the second amount of circulating tumor nucleic acid may be determined using cancer specific methylation markers present in circulating free nucleic acids in the blood of the patient. For example, gene sequencing, methylation specific gene sequencing, polymerase chain reaction (PCR), methylation specific PCR methods or other amplification methods and/or CRISPR based methods may be used to determine the first and/or second amount of circulating tumor nucleic acid. Alternatively, one or more mutation patterns in the circulating free nucleic acids may also be used to determine the first and second amount of circulating tumor nucleic acid.

The method additionally comprises computing a difference between the first amount of the circulating tumor nucleic acid and the second amount of the circulating tumor nucleic acid and determining the efficacy of the cancer therapy based on the computed difference. The second amount of the circulating tumor nucleic acid provides an indication of a change in the amount of circulating tumor nucleic acid in the patient's body. Advantageously, the method enables effective identification of efficacy of the cancer therapy early on, thereby saving time and cost associated with the treatment of the patient.

According to a preferred embodiment, the method further comprises defining a baseline associated with the circulating tumor nucleic acid in the patient, to determine the efficacy of the cancer therapy. The baseline may be defined, for example, based on the first amount of circulating tumor nucleic acid determined in the patient's body. Advantageously, the definition of the baseline enables accurate comparison of the amount of circulating tumor nucleic acid present in the blood of the patient, based on which the efficacy of the cancer therapy can be determined.

According to a further embodiment, the second amount of tumor nucleic acid is an estimation of tumor cell death caused by the cancer therapy, when the cancer therapy is ongoing. Therefore, determining the efficacy of the cancer therapy when the cancer therapy is ongoing comprises determining if the second amount of the circulating tumor nucleic acid is greater than the baseline. If the second amount of circulating tumor nucleic acid is greater than the baseline, an alert may be generated indicating that the efficacy of the cancer therapy is high. An increased second amount of circulating tumor nucleic acid may be an indication of increased tumor cell death that may result from the cancer therapy. Therefore, it can be said that the efficacy of the cancer therapy is high. However, if the second amount of circulating tumor nucleic acid is unchanged or below the baseline, an alert may be generated indicating that the efficacy of the cancer therapy is low. In an embodiment, the alert may be generated on an output unit of a user device associated with a physician, medical personnel and/or a hospital associated with the patient. In a further embodiment, the measurement of second amount of tumor nucleic acid and a comparative analysis of the second amount of tumor nucleic acid against the baseline may be performed multiple times during the progression of the cancer therapy. Advantageously, the alert provides the physician an early indication of the efficacy of the cancer therapy. Therefore, any modifications required in the treatment of the patient can be performed on a timely basis.

According to yet another embodiment, the second amount of circulating tumor nucleic acid may be an estimation of presence of tumor in the patient's body when the determination of the efficacy of the cancer therapy is performed after completion of the cancer therapy. Therefore, a determination is made if the second amount of the circulating tumor nucleic acid is equal to or greater than the baseline. If the second amount of circulating tumor nucleic acid is the same or greater than the baseline, an alert may be generated indicating that the efficacy of the cancer therapy is low. Such determination of the efficacy of the cancer therapy may be performed when the cancer therapy is completed. An increased second amount of circulating tumor nucleic acid may be an indication of recurrence of cancer in the patient after the therapy has been completed. Therefore, it can be said that the efficacy of the cancer therapy is low. However, if the second amount of circulating tumor nucleic acid is below the baseline, an alert may be generated indicating that the efficacy of the cancer therapy is high. In an embodiment, the alert may be generated on an output unit of a user device associated with a physician, medical personnel and/or a hospital associated with the patient. Advantageously, the alert provides the physician an effective indication of the efficacy of the cancer therapy for the patient. Therefore, any modifications required in the treatment of the patient can be performed on a timely basis.

According to another embodiment, the method further comprises determining a need for the patient to undergo medical imaging. Such determination may be made based on the efficacy of the cancer therapy. Therefore, depending on the outcome of the analysis of the cancer therapy efficacy, a decision may be taken if the medical imaging may be scheduled for the patient immediately or can be delayed. In an embodiment, a combination of circulating tumor nucleic acid analysis and medical imaging may be used to determine progress of the therapy for the patient. For example, in certain immunotherapy patients, medical imaging may indicate a growth in tumor size even though the tumor may be regressing. This may be identified as Pseudoprogression of the tumor. Therefore, the combination of circulating tumor nucleic acid analysis and medical imaging may enable distinguishing between Pseudoprogression and true progression of the tumor in the patient's body. Additionally, the amount of circulating tumor nucleic acid may also enable effective interpretation of findings from the medical imaging procedure.

In another aspect, the invention relates to a system for determining efficacy of a cancer therapy in a patient. The system includes one or more processing units, and a memory coupled to the one or more processing units. The memory further includes a module configured to perform the method steps as described above. In an alternate embodiment, the one or more processing units themselves may be configured to perform the method steps as described above.

In yet another aspect, the invention relates to a computer program product comprising machine readable instructions which when executed by one or more processing units causes the processing units to perform the method steps as described above.

In yet another aspect, the invention relate to a computer readable medium on which program code sections of a computer program are saved, the program code sections being loadable into and/or executable in a system to make the system execute the method steps as described above.

The present invention is further described hereinafter with reference to illustrated embodiments shown in the accompanying drawings, in which:
- FIG 1: illustrates a block diagram of a data processing system in which an embodiment for determining an efficacy of a cancer therapy in a patient can be implemented, according to an embodiment of the invention.
- FIG 2: illustrates a flowchart of a method of determining an efficacy of a cancer therapy in a patient, according to an embodiment of the invention.
- FIG 3: illustrates a flowchart of a method of determining the efficacy of the cancer therapy, according to a first embodiment of the invention.
- FIG 4: illustrates a flowchart of a method of determining the efficacy of the cancer therapy, according to a second embodiment.

Hereinafter, embodiments for carrying out the present invention are described in detail. The various embodiments are described with reference to the drawings, wherein like reference numerals are used to refer to like elements throughout. In the following description, for purpose of explanation, numerous specific details are set forth in order to provide a thorough understanding of one or more embodiments. It may be evident that such embodiments may be practiced without these specific details.

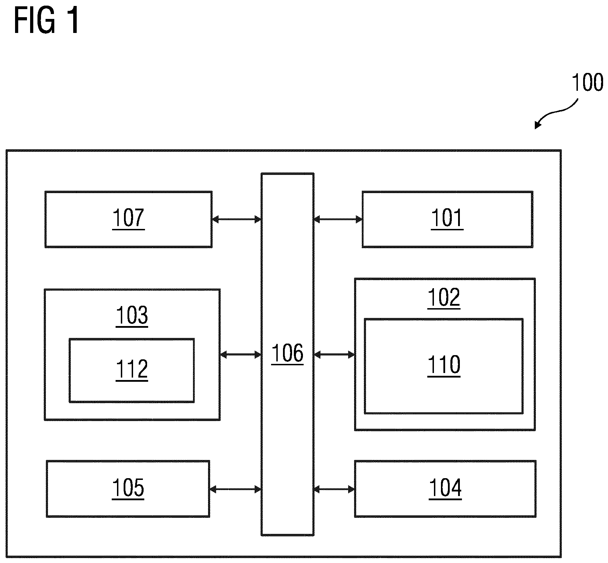

FIG 1 is a block diagram of a data processing system 100 in which an embodiment can be implemented, for example, as a system 100 for determining efficacy of a cancer therapy in a patient. In FIG 1, said data processing system 100 comprises a processing unit 101, a memory 102, a storage unit 103, an input unit 104, an output unit 105, a bus 106, and a network interface 107.

The processing unit 101, as used herein, means any type of computational circuit, such as, but not limited to, a microprocessor, microcontroller, complex instruction set computing microprocessor, reduced instruction set computing microprocessor, very long instruction word microprocessor, explicitly parallel instruction computing microprocessor, graphics processor, digital signal processor, or any other type of processing circuit. The processing unit 101 may also include embedded controllers, such as generic or programmable logic devices or arrays, application specific integrated circuits, single-chip computers, and the like.

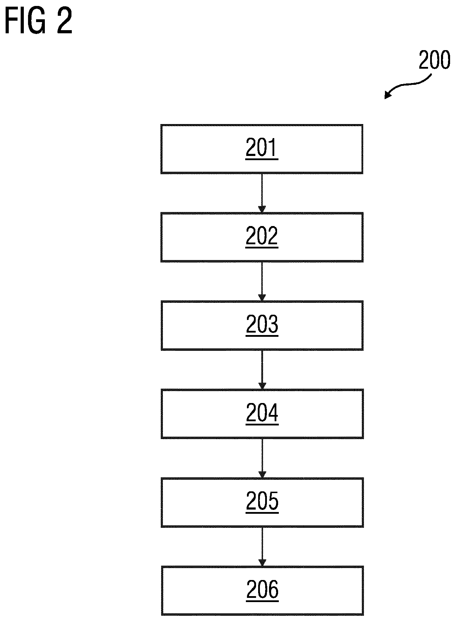

The memory 102 may be volatile memory and non-volatile memory. The memory 102 may be coupled for communication with said processing unit 101. The processing unit 101 may execute instructions and/or code stored in the memory 102. A variety of computer-readable storage media may be stored in and accessed from said memory 102. The memory 102 may include any suitable elements for storing data and machine-readable instructions, such as read only memory, random access memory, erasable programmable read only memory, electrically erasable programmable read only memory, a hard drive, a removable media drive for handling compact disks, digital video disks, diskettes, magnetic tape cartridges, memory cards, and the like. In the present embodiment, the memory 102 includes a module 110 stored in the form of machine-readable instructions on any of said above-mentioned storage media and may be in communication to and executed by processor 101. When executed by the processor 101, the module 110 causes the processor 101 to determine efficacy of a cancer therapy in a patient. In an alternate embodiment, the processing unit 101 itself may be configured to perform the method steps to determine efficacy of a cancer therapy in a patient. Method steps executed by the processor 101 to achieve the abovementioned functionality are elaborated upon in detail in FIG 2, 3 and 4.

The storage unit 103 may be a non-transitory storage medium which stores a database 112. The database 112 is a repository of amounts of circulating tumor nucleic acid determined in the patient. The input unit 104 may include input means such as keypad, touch-sensitive display, camera (such as a camera receiving gesture-based inputs), etc. capable of receiving input signal such as a medical image. The bus 106 acts as interconnect between the processor 101, the memory 102, the storage unit 103, the input unit 104, the output unit 105 and the network interface 107.

Those of ordinary skilled in the art will appreciate that said hardware depicted in FIG 1 may vary for particular implementations. For example, other peripheral devices such as an optical disk drive and the like, Local Area Network (LAN)/ Wide Area Network (WAN)/ Wireless (e.g., Wi-Fi) adapter, graphics adapter, disk controller, input/output (I/O) adapter also may be used in addition or in place of the hardware depicted. Said depicted example is provided for the purpose of explanation only and is not meant to imply architectural limitations with respect to the present disclosure.

A data processing system 100 in accordance with an embodiment of the present disclosure includes an operating system employing a graphical user interface. Said operating system permits multiple display windows to be presented in the graphical user interface simultaneously with each display window providing an interface to a different application or to a different instance of the same application. A cursor in said graphical user interface may be manipulated by a user through a pointing device. The position of the cursor may be changed and/or an event such as clicking a mouse button, generated to actuate a desired response.

One of various commercial operating systems, such as a version of Microsoft Windows^{™}, a product of Microsoft Corporation located in Redmond, Washington may be employed if suitably modified. Said operating system is modified or created in accordance with the present disclosure as described.

Disclosed embodiments provide systems and methods for determining efficacy of a cancer therapy in a patient.

FIG 2 illustrates a flowchart of a method 200 of determining efficacy of a cancer therapy in a patient, according to an embodiment of the invention. At step 201, the method includes determining a first amount of circulating tumor nucleic acid present in a blood sample obtained from the patient. Circulating tumor nucleic acid includes circulating tumor DNA (ctDNA) which can be identified based on unique signatures such as mutations or specific methylation patterns in the nucleic acid. In an embodiment, the circulating tumor nucleic acid also includes miRNA which may be dysregulated due to presence of cancer. Alternatively, circulating tumor nucleic acid also includes other molecules such as long non-coding RNA among other nucleic acids derived from cancer cells. The first amount of the circulating tumor nucleic acid provides an indication of an initial amount of circulating tumor nucleic acid present in the patient. In an embodiment, the circulating tumor nucleic acid may include circulating tumor DNA, micro RNA and/or long non-coding RNA associated with the tumor. Circulating tumor nucleic acid is obtained from a blood sample associated with the patient. Alternatively, the circulating tumor nucleic acid may also be obtained from samples such as serum, plasma, urine, saliva, and such other body fluids. Detection of the circulating tumor nucleic acid may be performed using gene sequencing, methylation specific gene sequencing, polymerase chain reaction (PCR), methylation specific PCR and/or other amplification methods. CRISPR based methods may also be used for determination of the circulating tumor nucleic acid in the patient's body.

At step 202, a baseline is defined based on the first amount of circulating tumor nucleic acid. The baseline forms a threshold with which subsequent measurements of circulating tumor nucleic acid may be compared. Therefore, the baseline enables effective identification of efficacy of the cancer therapy that the patient may undergo. At step 203, the patient is subjected to the cancer therapy. The therapy may be, but not be limited to, radiation therapy, chemotherapy and/or surgery. In an embodiment, the patient is subjected to at least one instance of the cancer therapy. At step 204, a second amount of circulating tumor nucleic acid present in the patient is determined after the patient is subjected to at least one instance of the cancer therapy. In an embodiment, the second amount of circulating tumor nucleic acid may change based on an effect of the cancer therapy on the tumor. At step 205, a difference between the first amount of circulating tumor nucleic acid and the second amount of circulating tumor nucleic acid is computed and at step 206, the efficacy of the cancer therapy is estimated based on the computed difference.

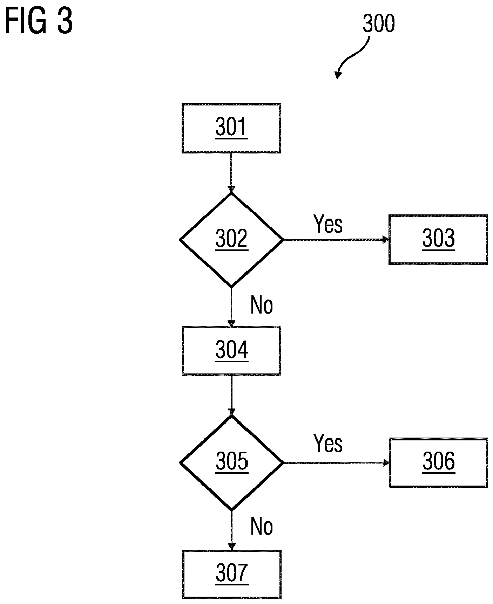

FIG 3 illustrates a flowchart of a method 300 of determining the efficacy of the cancer therapy, according to a first embodiment of the invention. In particular, the efficacy of the cancer therapy is determined during the pendency of the therapy, i.e. when the cancer therapy is ongoing. At step 301, the second amount of circulating tumor nucleic acid present in the patient's body is determined. Quantification of total amount of circulating tumor nucleic acid enables a physician to determine the efficacy of the cancer therapy. The circulating tumor nucleic acid is an indication of tumor cell death induced by the cancer therapy. At step 302, it is determined if the second amount of circulating tumor nucleic acid is greater than the baseline. If the second amount of circulating tumor nucleic acid is greater than the baseline, an alert is generated at step 304, indicating that the efficacy of the cancer therapy is high. However, if the amount of the circulating tumor nucleic acid is below the baseline, at step 305, it is determined if a medical image of the tumor is required to be obtained. The medical image may provide information to the physician regarding characteristics of the tumor. If the medical imaging is to be performed, a recommendation to perform the medical imaging for the patient is provided to the physician, at step 306. In an embodiment, the recommendation to perform medical imaging may be based on the second amount of circulating tumor nucleic acid present in the patient's body. Alternatively, the recommendation may also be based on an input received from the physician. At step 307, alternative cancer therapies may be recommended if the medical imaging is not required to be performed. Such recommendations may be based on tumor characteristics and other parameters associated with the patient such as patient demographic data.

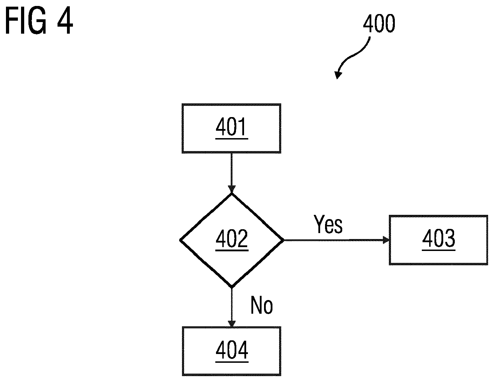

FIG 4 illustrates a flowchart of a method of determining the efficacy of the cancer therapy, according to a second embodiment of the invention. In particular, the efficacy of the cancer therapy is determined after the cancer therapy is completed. At step 401, the second amount of circulating tumor nucleic acid is determined after the cancer therapy is completed. At step 402, it is determined if the second amount of the circulating tumor nucleic acid is greater than the baseline. If found greater than the baseline, at step 403, an alert is generated indicating that the efficacy of the cancer therapy is low. A greater amount of circulating tumor nucleic acid in comparison the to the baseline may be an indication of presence of tumor in the patient's body, in spite of completion of the cancer therapy. Therefore, the cancer therapy may not have been effective in removing the tumor from the patient's body as predicted. If the second amount of circulating tumor nucleic acid is lower than the baseline, an alert is generated at step 404 indicating that the efficacy of the cancer therapy is high. This indicates an absence of/reduction in the size of tumor in the patient's body. In a further embodiment, a decision to obtain a medical image of the patient may also be made if the circulating tumor nucleic acid present in the patient's body is greater than the baseline.

The advantage of the invention is that early identification of ineffective cancer therapies is enabled. Additionally, the invention also enables early detection of relapses of cancer. Therefore, the life expectancy of patients is improved. Furthermore, morbidities associated with cancer therapy is avoided. The invention also enables better utilization of resources spent on treatment of the patient. The invention provides a sensitive method for effective determination of tumor cell death through measurement of circulating tumor nucleic acid.

The foregoing examples have been provided merely for the purpose of explanation and are in no way to be construed as limiting of the present invention disclosed herein. While the invention has been described with reference to various embodiments, it is understood that the words, which have been used herein, are words of description and illustration, rather than words of limitation. Further, although the invention has been described herein with reference to particular means, materials, and embodiments, the invention is not intended to be limited to the particulars disclosed herein; rather, the invention extends to all functionally equivalent structures, methods and uses, such as are within the scope of the appended claims. Those skilled in the art, having the benefit of the teachings of this specification, may effect numerous modifications thereto and changes may be made without departing from the scope and spirit of the invention in its aspects.

## Claims

1. A method (200) of determining efficacy of a cancer therapy in a patient, the method (200) comprising:
determining, by a processing unit (101), a first amount of circulating tumor nucleic acid present in the patient before initiating at least one instance of the cancer therapy;
determining, by the processing unit (101), a second amount of circulating tumor nucleic acid present in the patient after initiating the at least one instance of the cancer therapy;
computing, by the processing unit (101), a difference between the first amount of circulating tumor nucleic acid and the second amount of circulating tumor nucleic acid; and
estimating, by the processing unit (101), the efficacy of the cancer therapy based on the computed difference.

2. The method (200) according to claim 1, wherein determining the efficacy of the cancer therapy comprises: defining a baseline associated with the circulating tumor nucleic acid and comparing the second amount to the baseline.

3. The method (200) according to 2, wherein the baseline is based on the first amount of circulating tumor nucleic acid in the patient.

4. The method (200) according to any one of claims 1 - 3, wherein the second amount of circulating tumor nucleic acid is an estimation of tumor cell death caused by the cancer therapy and wherein the determination of the efficacy of the cancer therapy is performed when the cancer therapy is ongoing.

5. The method (200) according to any one of claims 2 - 4,
if the second amount of circulating tumor nucleic acid is greater than the baseline, generating an alert indicating that the efficacy of the cancer therapy is high; and/or
if the second amount of circulating tumor nucleic acid is lower than or similar to the baseline, generating an alert indicating that the efficacy of the cancer therapy is low.

6. The method (200) according to any one of claims 1 - 5, wherein the second amount of circulating tumor nucleic acid is an estimation of presence of tumor in the patient's body and wherein the determination of the efficacy of the cancer therapy is performed after completion of the cancer therapy.

7. The method (200) according to any one of claims 2 - 6, wherein:
if the second amount of circulating tumor nucleic acid is greater than the baseline, generating an alert indicating that the efficacy of the cancer therapy is low; and/or
if the second amount of circulating tumor nucleic acid is lower than or similar to the baseline, generating an alert indicating that the efficacy of the cancer therapy is high.

8. The method (200) according to any one claim 1 - 7, further comprising determining based on the efficacy of the cancer therapy if there exists a need for the patient to undergo medical imaging.

9. The method (200) according to any of the aforementioned claims, wherein the circulating tumor nucleic acid is determined based on a unique signature associated with the circulating tumor nucleic acid.

10. The method (200) according to any of the aforementioned claims, wherein the first and/or second amount of circulating tumor nucleic acid is determined using samples of the patient such as blood, serum, plasma, urine, saliva or other body fluids.

11. The method (200) according to any of the aforementioned claims, wherein the first and/or second amount of circulating tumor nucleic acid is determined using sequencing, or methylation specific sequencing or PCR or methylation specific PCR or other amplification or CRISPR based methods.

12. A system (100) for determining efficacy of a cancer therapy in a patient, the system (100) comprising:
one or more processing units (101) configured to perform a method (200) as claimed in claims 1 to 11.

13. A computer program product comprising machine readable instructions, that when executed by one or more processing units, cause the one or more processing units (101) to perform method steps according to claims 1 to 11.

14. A computer readable medium on which program code sections of a computer program are saved, the program code sections being loadable into and/or executable in a system (100) to make the system (100) execute the method steps according to any one of the claims 1 to 11 when the program code sections are executed in the system (100).
